# EUROPEAN PATENT APPLICATION

(11) **EP 4 620 395 A1**
(43) Date of publication of application: **24.09.2025**
(21) Application number: 25165477.8
(22) Date of filing: 21.03.2025
(51) Int. Cl.: A61B 5/339, A61B 5/349, A61B 5/00

(54) **COMPUTERIZED ECG INTERPRETATION VISUALIZATION**

(30) Priority: 22.03.2024 US 202463568546 P; 19.03.2025 US 202519083634
(71) Applicant: Welch Allyn, Inc., Skaneateles Falls, NY 13153-0220 (US)
(72) Inventor: Noffke, Patrick James, Skaneateles Falls, 13153 (US); Donlon, Ryan, Skaneateles Falls, 13153 (US); Khawar, Waqaar, Skaneateles Falls, 13153 (US); Marzocchi, Nicoletta, Skaneateles Falls, 13153 (US); Zlochiver, Sharone, Skaneateles Falls, 13153 (US)
(74) Representative: Reddie & Grose LLP

(57) **Abstract**

An electrocardiogram interpretation system includes a display and a user interface. A processor receives an ECG that includes a waveform of electrical activity of a patient's heart over a period of time and analyzes the waveform to calculate computerized interpretation metrics and detect at least one segment of the waveform corresponding to a behavior associated with a condition of the patient's heart and categorize a type of the condition and a status of the condition selected from a group including at least normal and abnormal with the computerized interpretation metrics. The processor generates, on the display, the waveform and highlight the at least one segment with a color selected from a plurality of colors, each color of the plurality of colors corresponding to the type of condition or the status of the condition. The processor generates, on the display, textual information that includes the type of condition or the status of the condition.

## Description

Computerized electrocardiography ("ECG") interpretation has been in development for several decades. As technology advances, various devices have been developed with computerized interpretation ("CI"), such as electrocardiographs, which analyze the ECG and present measurements (e.g., HR, PR, QRS duration, QT/QTc) and textual interpretation statements. The results of the CI are unconfirmed, assistive in nature, and must be reviewed by a physician in order for them to be clinically meaningful. In practice, however, physicians may rely on the Cl to make clinical decisions. As such, it is important that a physician understand the basis of the Cl in order to accurately and independently make a clinically meaningful diagnosis.

Accordingly, there is a continued need to make not only the CI understandable to physicians, but also the basis for the Cl, such that the physician can accurately and independently form a diagnosis.

According to one aspect of the present disclosure, an electrocardiogram ("ECG") interpretation system includes a display, a user interface, a processor, and a memory. The memory contains instructions that, when executed by the processor, cause the processor to receive an ECG that includes a waveform of electrical activity of a patient's heart over a period of time and analyze the waveform to calculate computerized interpretation metrics. The processor is further caused to detect at least one segment of the waveform corresponding to a behavior associated with a condition of the patient's heart and categorize a type of the condition and a status of the condition selected from a group including at least normal and abnormal with the computerized interpretation metrics. The processor is further caused to generate, on the display, the waveform and highlight the at least one segment with a color selected from a plurality of colors, each color of the plurality of colors corresponding to the type of condition or the status of the condition. The processor is further caused to generate, on the display, textual information that includes the type of condition or the status of the condition.

According to another aspect of the present disclosure, an electrocardiogram ("ECG") interpretation system includes a display, a user interface, a processor, and a memory. The memory contains instructions that, when executed by the processor, cause the processor to receive an ECG that includes a waveform of electrical activity of a patient's heart over a period of time and analyze the waveform to calculate computerized interpretation metrics. The processor is further caused to detect at least one segment of the waveform corresponding to an abnormality of the patient's heart with the computerized interpretation metrics. The processor is further caused to generate, on the display, the waveform, and generate, on the display, textual information that includes the type of the abnormality and highlight the textual information with a color selected from a plurality of colors, each color of the plurality of colors corresponding to the severity of the abnormality.

According to yet another aspect of the present disclosure, an electrocardiogram ("ECG") interpretation system includes a display, a user interface, a processor, and a memory. The memory contains instructions that, when executed by the processor, cause the processor to receive an ECG that includes a waveform of electrical activity of a patient's heart over a period of time and analyze the waveform to calculate computerized interpretation metrics. The processor is further caused to detect at least one segment of the waveform corresponding to a behavior associated with a condition of the patient's heart and categorize a type of the condition and a status of the condition selected from a group including at least normal and abnormal with the computerized interpretation metrics. The processor is further caused to generate, on the display, the waveform and highlight the at least one segment with a color selected from a plurality of colors, each color of the plurality of colors corresponding to the type of condition or the status of the condition. The processor is further caused to generate, on the display, textual information that includes the type of condition or the status of the condition and highlight the textual information with the color selected from the plurality of colors, each color of the plurality of colors corresponding to the type of condition or the status of the condition.

According to still yet another aspect of the disclosure, an electrocardiogram ("ECG") interpretation system includes a display, a user interface, a processor, and a memory. The memory contains instructions that, when executed by the processor, cause the processor to receive an ECG that includes a waveform of electrical activity of a patient's heart over a period of time and analyze the waveform to calculate computerized interpretation metrics. The processor is further caused to detect at least one segment of the waveform corresponding to a behavior associated with a condition of the patient's heart and categorize a type of the condition and a status of the condition selected from a group including at least normal and abnormal with the computerized interpretation metrics. The processor is further caused to generate, on the display, the waveform and highlight the at least one segment with a first color selected from a plurality of first colors, each first color corresponding to the type of condition or the status of the condition. The processor is further caused to generate, on the display, textual information that includes the type of condition or the status of the condition and highlight the textual information with the first color selected from the first plurality of colors, each color of the plurality of colors corresponding to the type of condition or the status of the condition.

According to still another aspect of the disclosure, an electrocardiogram ("ECG") interpretation system includes an electrocardiogram device configured to place over a patient's heart that generates a cardiac signal. The ECG interpretation system further includes a display, a user interface, a processor, and a memory. The memory contains instructions that, when executed by the processor, cause the processor to receive, from the electrocardiogram device, the cardiac signal and generate an ECG that includes a waveform of electrical activity of a patient's heart over a period of time, and analyze the waveform to calculate computerized interpretation metrics. The processor is further caused to detect at least one segment of the waveform corresponding to a behavior associated with a condition of the patient's heart and categorize a type of the condition and a status of the condition selected from a group including at least normal and abnormal with the computerized interpretation metrics. The processor is further caused to generate, on the display, the waveform and highlight the at least one segment with a color selected from a plurality of colors, each color of the plurality of colors corresponding to the type of condition or the status of the condition. The processor is further caused to generate, on the display, textual information that includes the type of condition or the status of the condition.

According to still yet another aspect, a method of interpreting an electrocardiogram ("ECG") includes receiving an ECG that includes a waveform of electrical activity of a patient's heart over a period of time. The waveform is analyzed to calculate computerized interpretation metrics and at least one segment of the waveform is detected that corresponds to a behavior associated with a condition of the patient's heart. The method further includes categorizing a type of the condition and a status of the condition selected from a group including at least normal and abnormal with the computerized interpretation metrics, and generating, on a display, the waveform and highlight the at least one segment with a color selected from a plurality of colors, each color of the plurality of colors corresponding to the type of condition or the status of the condition. On the display, textual information is generated that includes the type of the-condition and the status of the condition.

These and other features, advantages, and objects of the present disclosure will be further understood and appreciated by those skilled in the art by reference to the following specification, claims, and appended drawings.

The invention will now be further described by way of example with reference to the accompanying drawings, in which:
FIG. 1 is a partially schematic view of an electrocardiogram interpretation system that includes a control system, a display, and an electrocardiogram device, according to the present disclosure;
FIG. 2 is a schematic view of a control system of an electrocardiogram interpretation system, according to the present disclosure;
FIG. 3 is a first enlarged image on a display generated by a control system of an electrocardiogram interpretation system, according to the present disclosure;
FIG. 4 is a second enlarged image on a display generated by a control system of an electrocardiogram interpretation system, according to the present disclosure;
FIG. 5 is a third enlarged image on a display generated by a control system of an electrocardiogram interpretation system, according to the present disclosure;
FIG. 6 is a fourth enlarged image on a display generated by a control system of an electrocardiogram interpretation system, according to the present disclosure;
FIG. 7 is a fifth enlarged image on a display generated by a control system of an electrocardiogram interpretation system, according to the present disclosure;
FIG. 8 is an image on a display generated by a control system of an electrocardiogram interpretation system, according to the present disclosure;
FIG. 9 is a first partial image on a display generated by a control system of an electrocardiogram interpretation system, according to the present disclosure;
FIG. 10 is a second partial image on a display generated by a control system of an electrocardiogram interpretation system, according to the present disclosure;
FIG. 11 is a third partial image on a display generated by a control system of an electrocardiogram interpretation system, according to the present disclosure;
FIG. 12 is a fourth partial image on a display generated by a control system of an electrocardiogram interpretation system, according to the present disclosure; and
FIG. 13 is a flow chart showing a method of visualizing a computerized interpretation on a display with a user interface, according to the present disclosure.

The present illustrated embodiments reside primarily in combinations of method steps, systems, devices, and apparatus components related to a visualization system that assists a caregiver in interpreting an electrocardiogram by highlighting points of interest. Accordingly, the apparatus components and method steps have been represented, where appropriate, by conventional symbols in the drawings, showing only those specific details that are pertinent to understanding the embodiments of the present disclosure so as not to obscure the disclosure with details that will be readily apparent to those of ordinary skill in the art having the benefit of the description herein. Further, like numerals in the description and drawings represent like elements.

The specific structures and processes illustrated in the attached drawings, and described in the following specification are simply exemplary embodiments of the inventive concepts defined in the appended claims. Hence, specific dimensions and other physical characteristics relating to the embodiments disclosed herein are not to be considered as limiting, unless the claims expressly state otherwise.

The terms "including," "comprises," "comprising," or any other variation thereof, are intended to cover a non-exclusive inclusion, such that a process, method, article, or apparatus that comprises a list of elements does not include only those elements but may include other elements not expressly listed or inherent to such process, method, article, or apparatus. An element preceded by "comprises a . . . " does not, without more constraints, preclude the existence of additional identical elements in the process, method, article, or apparatus that comprises the element.

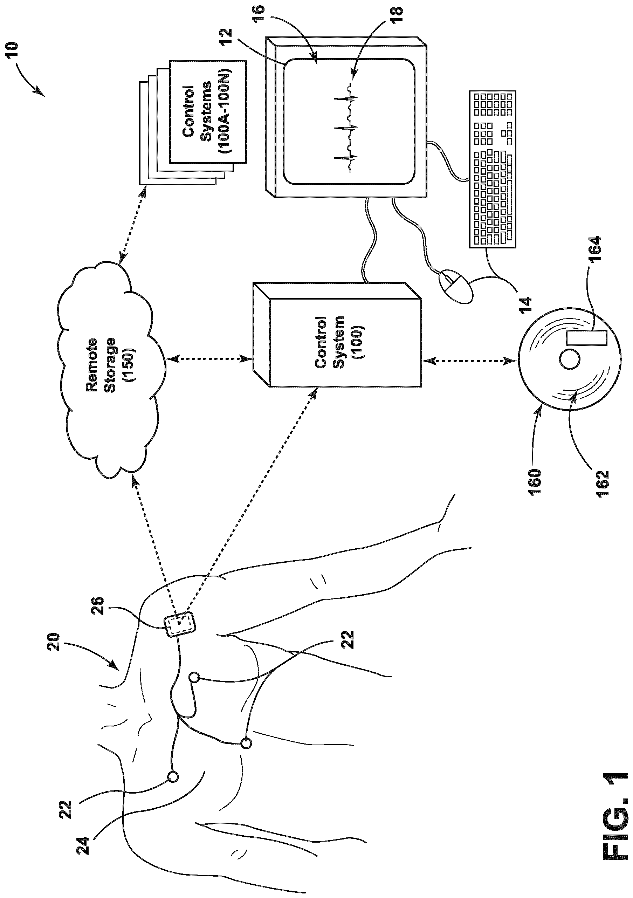

Referring initially to FIGS. 1-9, reference numeral 10 designates an electrocardiogram ("ECG") interpretation system. The ECG interpretation system 10 includes a display 12, a user interface 14, a control system 100 including a processor 104, and a memory 106. The memory 106 contains instructions that, when executed by the processor 104, cause the processor 104 to receive an ECG 16 that includes a waveform 18 of electrical activity of a patient's heart over a period of time and analyze the waveform 18 to calculate computerized interpretation metrics. The processor 104 then detects at least one segment S1 of the waveform 18 corresponding to a behavior associated with a condition of the patient's heart and categorizes the type of condition or the status of the condition with the computerized interpretation metrics. The processor 104 then generates, on the display 12, the waveform 18 and highlights the at least one segment S1 with a color selected from a plurality of colors, each color of the plurality of colors corresponding to the type of condition or the status of the condition. The processor 104 further generates, on the display 12, textual information "Ti" that includes the type of condition or the status of the condition.

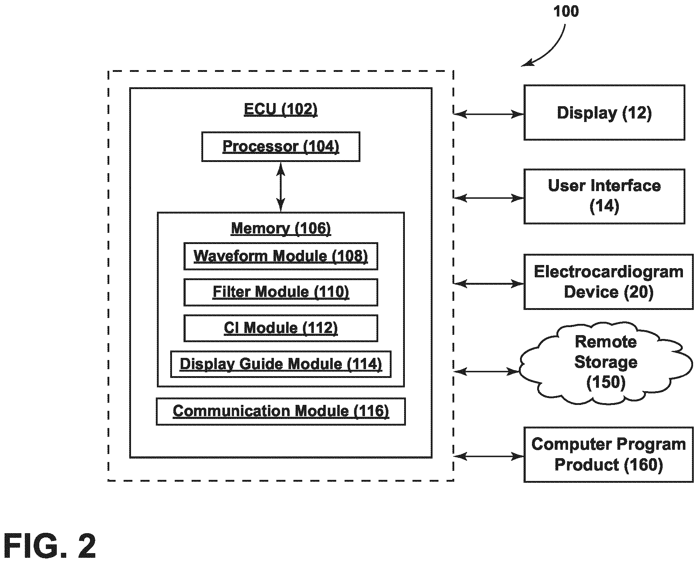

Referring now to FIGS. 1 and 2, the ECG interpretation system 10 may receive the ECG 16 from an electrocardiogram device 20 directly or indirectly. For example, in some embodiments, the ECG interpretation system 10 (e.g., the control system 100) may be configured to receive, from the electrocardiogram device 20, a cardiac signal and generate the ECG 16 that includes the waveform 18 of electrical activity of the patient's heart. The electrical activity of the patient's heart may correspond to cardiac muscle activity, non-cardiac muscle activity, motion artifacts, combinations thereof, and/or the like. In such embodiments, the comparison to the computerized interpretation metrics may occur in real-time and/or near real-time for monitoring a current condition of the patient's heart. Further in such embodiments, the controller 100 may be configured to control functionality of both the electrocardiogram device 20 (i.e., the cardiac signal generating component) and the computerized interpretation components. However, in other embodiments, the electrocardiogram device 20, or a computing device different than the electrocardiogram device 20 and the controller 100, may be configured to generate the ECG 16 from the cardiac signal that is stored remotely or locally which can later be received by the control system 100 (e.g., memory 106) for comparison to the computerized interpretation metrics.

With reference to FIG. 1, the electrocardiogram device 20 may be configured to place over the patient's heart. More particularly, the electrocardiogram device 20 may include a plurality of electrodes 22 placed on a patient's chest 24 proximate the patient's heart for generating the cardiac signal that is used to generate the waveform 18 over a period of time. The electrocardiogram device 20 may include a communication device 26 in communication with at least one of the control system 100 and the remote storage 150. The communication may be wired or wireless. It should be appreciated that the electrocardiogram device 20 depicted in FIG. 1 is exemplary only, electrocardiogram devices of different configurations may be utilized for receiving and/or generating the cardiac signal. For example, electrocardiogram devices that are mobile, stationary, and operating under various principles with different configurations of electrodes may be used other than the electrocardiogram device 20 depicted in FIG. 1. For example, in some embodiments, the electrodes 22 may be in a 12-lead configuration. In use, the behavior (e.g., electrical activity) of the patient's heart generates the cardiac signal, which is transmitted to the control system 100. The control system 100 may store the cardiac signal locally and/or in a remote storage 150, such as a cloud or a remote server. The remote storage 150 may be operably connected to the control system 100 and numerous additional control systems 100A-100N located within a medical environment (e.g., a hospital or medical campus) of the electrocardiogram device 20, locally, regionally, nationally, or internationally. The control system 100 accesses the cardiac signal (e.g., electrical activity) to generate the waveform 18 over the period of time and analyzes the waveform 18 to calculate computerized interpretation metrics, wherein different features can be accessed via the display 12. The display 12 may be configured as a computer, a tablet, and/or the like and include a user interface 14. The user interface 14 may include a touch screen, a keypad (e.g., a keyboard), a mouse and/or the like for interacting with the visualization on the display 12.

FIG. 2 schematically illustrates the control system 100. The control system 100 may include an electronic control unit (ECU) 102. The ECU 102 may include the processor 104 and the memory 106. The processor 104 may include any suitable processor 104. Additionally, or alternatively, the ECU 102 may include any suitable number of processors, in addition to or other than the processor 104. The memory 106 may comprise a single disk or a plurality of disks (e.g., hard drives) and includes a storage management module that manages one or more partitions within the memory 106. In some embodiments, memory 106 may include flash memory, semiconductor (solid-state) memory, or the like. The memory 106 may include Random Access Memory (RAM), a Read-Only Memory (ROM), Electrically Erasable Programmable Read-Only Memory (EEPROM), or a combination thereof. The memory 106 may include instructions that, when executed by the processor 104, cause the processor 104 to, at least, perform the functions associated with the components of the control system 100. The electrocardiogram device 20, the display 12, and the user interface 14 may therefore be controlled and/or receive instructions from the ECU 102. In some embodiments, the electrocardiogram device 20 may include a control system different than the control system 100. The memory 106 may therefore include a waveform module 108, a filter module 110, a computerized interpretation module 112 ("Cl module"), and a display guide module 114. The control system 100 may further include a communication module 116 that receives/transmits information with the remote storage 150 and/or the electrocardiogram device 20.

With continued reference to FIG. 2, the control system 100 is configured to receive at least one of the cardiac signal or the waveform 18 over the period of time from the electrocardiogram device 20 directly or from the remote storage 150. When only the cardiac signal is received, the waveform module 108 may generate the waveform 18 and save the waveform in memory 106 (e.g., in the waveform module 108). When the waveform 18 is received from electrocardiogram device 20 or the remote storage 150, the waveform 18 may be stored in the waveform module 108. Once the waveform 18 is received. The filter module 110 may detect anomalies from the waveform 18 (i.e., incorrect electrode 22 placement, shifting of the patient's chest 24, etc.). The anomalies may still be shown in the waveform 18 but the control system 100 (e.g., the processor 104) may generate a textual note or other indicia so that a healthcare provider can make a final judgment on whether to consider the anomalies in the clinical diagnosis. The waveform 18 is then compared to the computerized interpretation metrics, which may be located in the computerized interpretation module 112 and/or in the remote storage 150. The computerized interpretation module 112 may include a library of predictive models, threshold values, and other information metrics related to a normal waveform 18, including threshold values of the waveform 18 corresponding to the type of condition or the status of the condition (e.g., a severity and/or abnormality type). More particularly, the computerized interpretation module 112 may operate under the principles of various commercial products that provide computerized interpretation metrics. Based on the comparison between the waveform 18 and computerized interpretation metrics, the control system 100 (e.g., the processor 104 receiving instructions from the computerized interpretation module 112) is further caused to detect at least one segment S1 of the waveform corresponding to the type of condition or the status of the condition of the patient's heart and categorize the type of condition or the status of the condition. The control system 100 (e.g., the processor 104 receiving instructions from the display guide module 114) is further caused to generate, on the display 12, the waveform 18 and highlight the at least one segment S1 with the color. The display guide module 114 may include the plurality of colors, with each color of the plurality of colors corresponding to the type of condition or the status of the condition (e.g., the presence and severity of an abnormality or normal behavior). The control system 100 (e.g., the processor 104 receiving instructions from the display guide module 114) is further caused to generate, on the display 12, textual information Ti that includes the type of condition or the status of the condition.

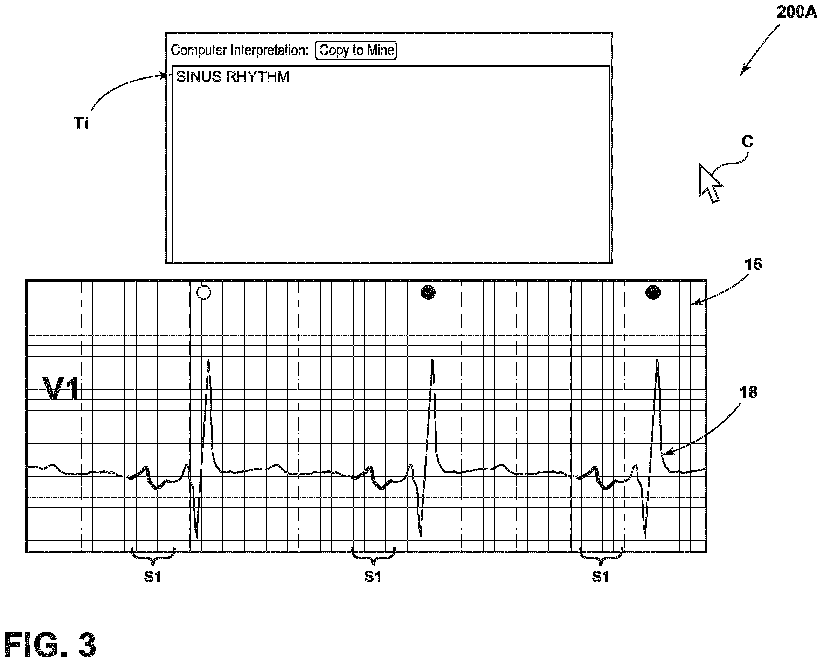

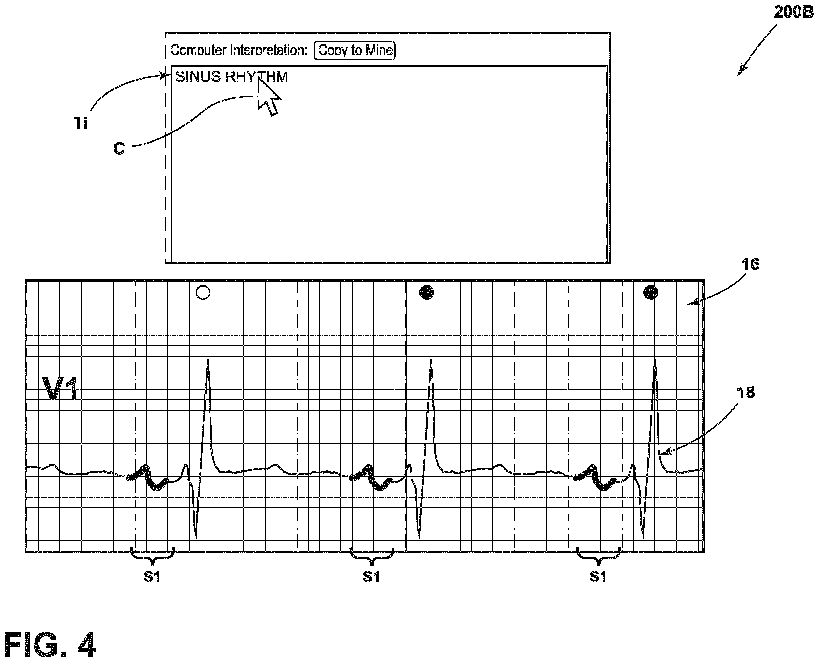

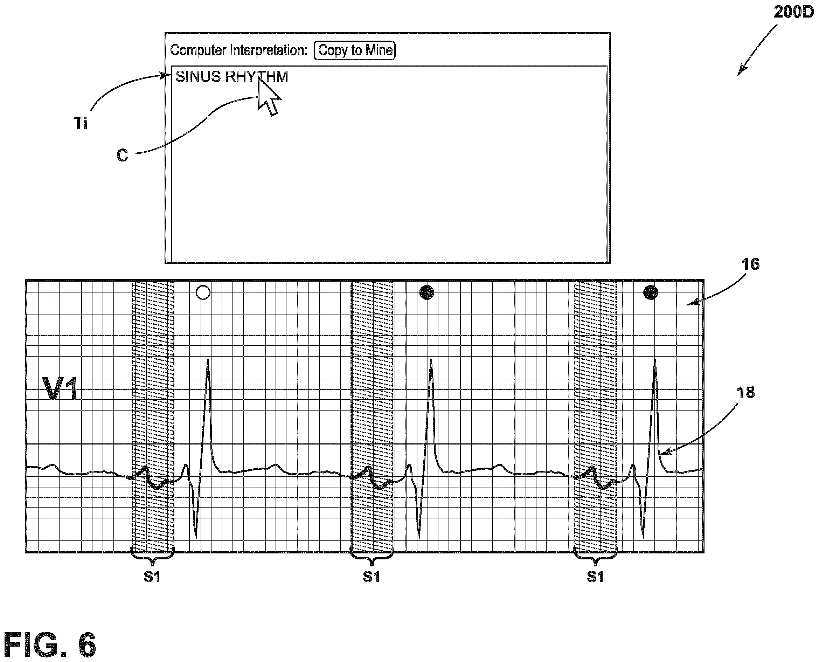

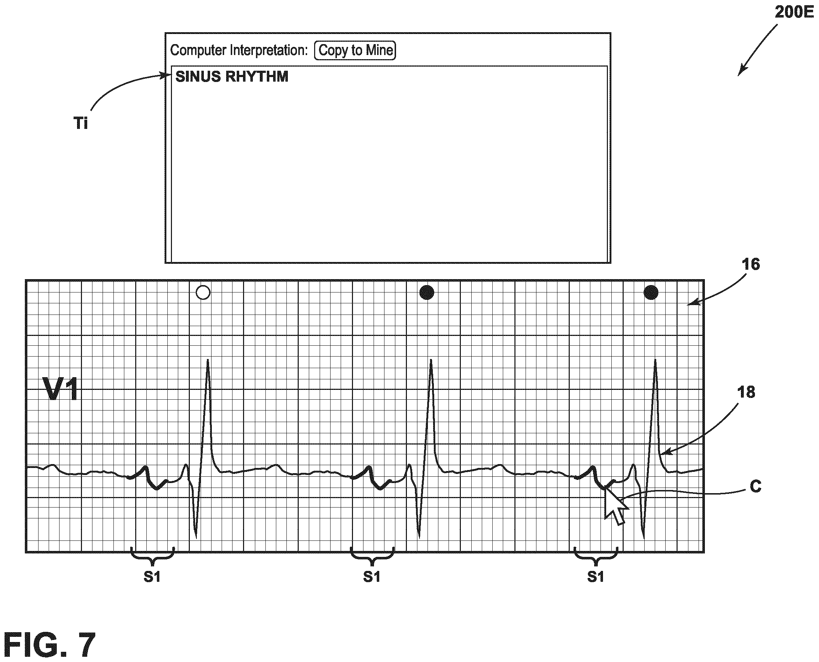

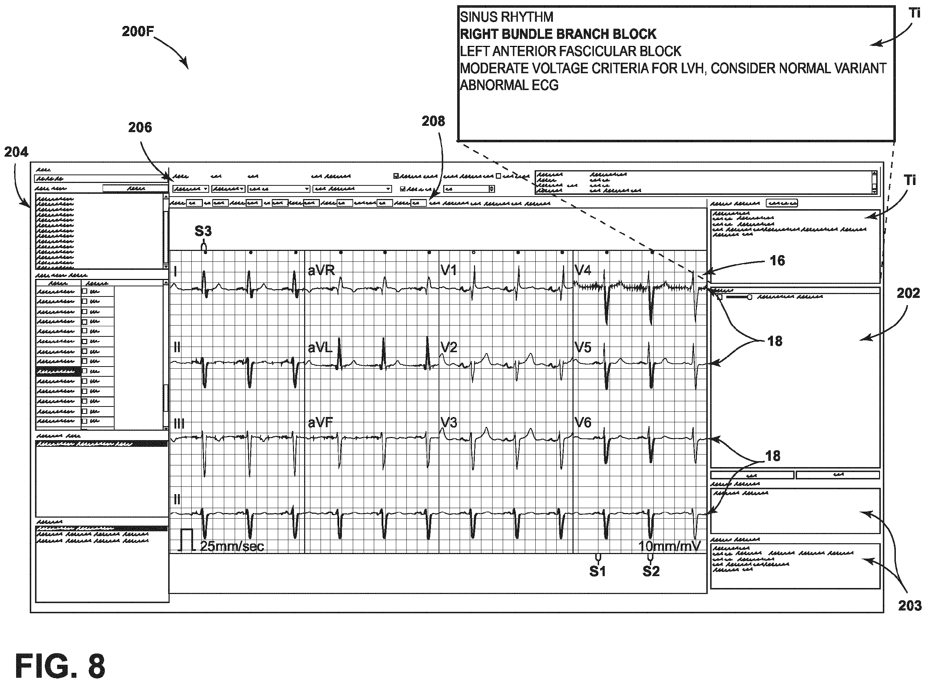

FIGS. 3-12 illustrate various examples of the images 200A-200J generated on the display 12. However, it should be appreciated that the images 200A-200J are exemplary in nature, and a variety of alternative ways of visualizing important information could be utilized. For example, the term highlight may be defined as a generation of an indicia on the image 200A-200J to draw the healthcare provider's attention to the at least one segment S1 that is abnormal and to visualize the type of condition or the status of the condition associated with the at least one segment S1. The highlight may include selecting a color out of a plurality of colors based on severity. In some embodiments, the color may be generated on the segment S1, such that the waveform 18 is the color. In some embodiments, the color may be generated on a background behind the segment S1 (FIG. 6). Likewise, a matching highlight (e.g., the same color) may be generated relative to the textual information Ti. In some embodiments, the color may be generated on the textual information, such that font is the color. In some embodiments, the color may be generated on a background behind the textual information. The highlight may include other types of indicia, for example, the highlight may include bolding/patterning the waveform 18 or the font of the textual information Ti (FIGS. 4 and 7). In some embodiments, two or more highlights may be utilized (FIGS. 8-12). For example, a first type of highlight corresponding to the type of condition and a second type of highlight corresponding to the status of the condition (e.g., abnormality presence and severity). In some embodiments, the two highlights may be applied to the segment S1 and/or the textual information Ti. In still other embodiments, the first highlight may be generated on one of the segments S1-SN or the textual information Ti and the second highlight may be generated on the other of the segment S1-SN and the textual information Ti. In addition to the highlight, it should be appreciated that the textual information Ti may include the type and or status of the condition in written form.

With reference now to FIGS. 1-12, in some embodiments, the control system 100 (e.g., the processor 104) may be configured to, based on instructions in the memory 106 or remote storage 150, receive an ECG 16 that includes a waveform 18 of electrical activity of a patient's heart over a period of time. For example, the ECG 16 may be received from the electrocardiogram device 20 or from the remote storage 150 and may further be stored in memory 106 (i.e., the waveform module 108).

With reference now to FIGS. 2-12, in some embodiments, the control system 100 (e.g., the processor 104) may be configured to, based on instructions in the memory 106 or remote storage 150, detect anomalies (i.e., with the filter module 110) from the waveform 18 (i.e., incorrect electrode 22 placement, shifting of the patient's chest 24, etc.). The anomalies may still be shown in the waveform 18 but the control system 100 (e.g., the processor 104) may generate a textual note or other indicia so that a healthcare provider can make a final judgment on whether to consider the anomalies in the clinical diagnosis. It should be appreciated that, in some embodiments, the filtering operation by the filtering module 110 may be included in the computerized interpretation module 112 (e.g., with predictive models, threshold information, and/or the like).

With reference now to FIGS. 3-12, in some embodiments, the control system 100 (e.g., the processor 104) may be configured to, based on instructions in the memory 106 or remote storage 150, analyze the waveform 18 to calculate computerized interpretation metrics. For example, the memory 106 (i.e., the computerized interpretation module 112) or remote storage 150. The computerized interpretation module 112 and/or remote storage 150 may include a library of predictive models, threshold values, and other information metrics related to a normal waveform 18, including threshold values of the waveform 18 corresponding to the type of condition or the status of the condition.

With continued reference to FIGS. 3-12, in some embodiments, the control system 100 (e.g., the processor 104) may be configured to, based on instructions in the memory 106 or remote storage 150, detect at least one segment S1 of the waveform 18 corresponding to a behavior associated with a condition of the patient's heart and categorize the type of condition or the status of the condition with the computerized interpretation metrics. For example, the computerized interpretation module 112 and/or remote storage 150 may include a library of predictive models, threshold values, and other information metrics related to a normal waveform 18, including threshold values of the waveform 18 corresponding to the type of condition or the status of the condition.

With reference now to FIGS. 3-7, an enlarged view of various portions of images 200A-200E on a display 12 are illustrated. In some embodiments, the control system 100 (e.g., the processor 104) may be configured to, based on instructions in the memory 106 (i.e., the display guide module 114) or remote storage 150, generate on the display 12, the waveform 18 and highlight the at least one segment S1 with a color selected from a plurality of colors, each color of the plurality of colors corresponding to the type of condition or the status of the condition. The processor 104 is further caused to generate, on the display 12, textual information "Ti" that includes the type of the condition. For example, the enlarged image 200A depicted in FIG. 3 shows the waveform 18 and the highlight to the at least one segment S1 and the textual information Ti. In some embodiments, the computerized interpretation metrics in the computerized interpretation module 112 may include different types of highlights (i.e., color, line type, pattern, font type) corresponding to different levels of the status of the condition. The textual information Ti may include information related to portions of the waveform 18 other than the at least one segment S1. Indeed, it should be appreciated that the at least one segment S1 could include a plurality of segments S1-SN corresponding to the same detected type of condition or multiple different types of conditions. In instances with detected multiple different types of conditions, the highlight may, in addition to visualizing status (e.g., severity), visualize the type of condition (i.e., with two or more of the plurality of colors, the textual information Ti, line type, line size, font size, and/or the like). As will be appreciated with further reading, the highlight may be automatically generated on the display 12 or may otherwise only be generated based on inputs from the user interface 14. In some embodiments, the textual information Ti that includes the type of the condition is highlighted in the color of the highlight to the at least one segment S1.

With continued reference to FIGS. 3-7, in some embodiments, the control system 100 (e.g., the processor 104) may be configured to, based on instructions in the memory 106 (i.e., the display guide module 114) or remote storage 150, highlight the waveform 18 (e.g., the at least one segment S1) by colorizing the at least one segment in the color, such as the waveform 18 in the enlarged image 200A depicted in FIG. 3. In some embodiments, the highlight on the textual information includes colorizing the font of the textual information Ti in the color (i.e., the same color as the segment S1).

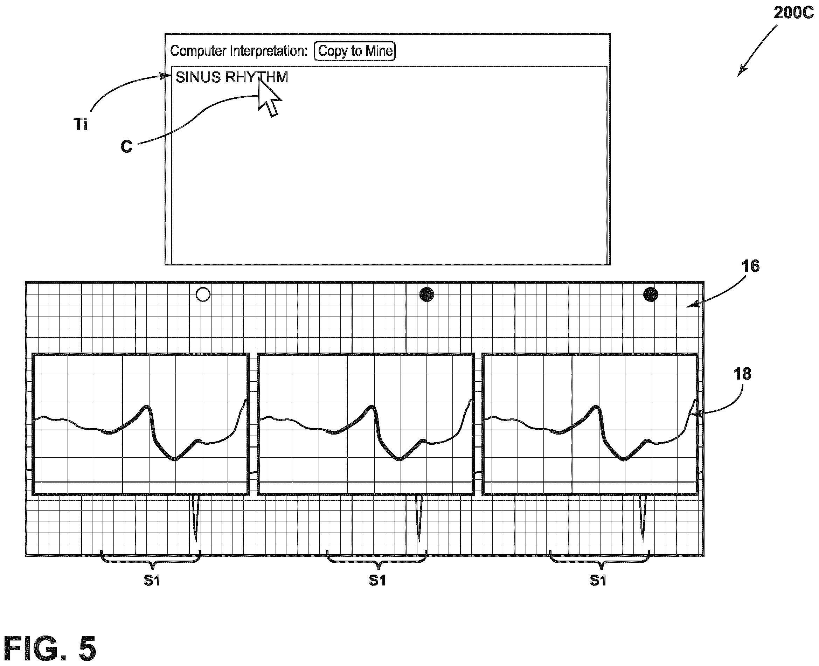

With reference now to FIGS. 4-6, in some embodiments, the user interface 14 includes a cursor and the control system 100 (e.g., the processor 104) may be configured to, based on instructions in the memory 106 (i.e., the display guide module 114) or remote storage 150, detect if the cursor is aligned with the textual information Ti that includes the type of the condition and enlarge the at least one segment S1 on the display 12. For example, in the waveform 18 in enlarged image 200B depicted in FIG. 4, when the cursor is aligned with the textual information Ti, the at least one segment S1 is thickened. Similarly, in the waveform 18 in enlarged image 200C depicted in FIG. 5, when the cursor is aligned with the textual information Ti, the at least one segment S1 is zoomed in on. In cases where there are numerous segments S1-SN associated with the type of condition and/or the status of the condition, each or select segments S1-SN may be zoomed in on and/or thickened. In this manner, a healthcare provider reviewing the ECG 16 may quickly see which segments S1-SN formed the basis for the categorization of the type of condition or the status of the condition with the computerized interpretation metrics in order to make a clinical diagnosis. In other embodiments, such as the waveform 18 in the enlarged image 200D depicted in FIG. 6, the segment S1 may be highlighted by colorizing a background of the segment S1. Likewise, in other embodiments, the textual information Ti may be highlighted by colorizing a background behind the font.

With reference now to FIG. 7, in some embodiments, the user interface 14 includes the cursor and the control system 100 (e.g., the processor 104) may be configured to, based on instructions in the memory 106 (i.e., the display guide module 114) or remote storage 150, detect if the cursor on the enlarged image 200E is aligned with the at least one segment S1 and enlarge textual information Ti that includes the type of the condition. For example, the textual information Ti may be bolded and/or provided in a larger font. In this manner, a healthcare provider reviewing the ECG 16 may quickly see which segments S1-SN correspond to the textual information Ti in order to make a clinical diagnosis.

With continued reference to the image 200E depicted in FIG. 7, in some embodiments, the control system 100 (e.g., the processor 104) may be configured to, based on instructions in the memory 106 (i.e., the display guide module 114) or remote storage 150, initially generate, on the display 12, textual information Ti that includes the type of the condition and highlight the textual information Ti with a color selected from a plurality of colors, rather than or in addition to the segments S1-SN. In some embodiments, the segment S1-SN is not initially colored until the specific textual information Ti is selected with the user interface 14 (e.g., the cursor), where the associated segments S1-SN become colored. In some embodiments, both the textual information Ti and the segments S1-SN are initially covered. In some embodiments, only the segments S1-SN are initially colored, and the textual information Ti is not colored or becomes colored based on the selection of the segment S1-SN with the user interface 14 (e.g., the cursor).

With reference now to FIGS. 8-12, the highlight may include the plurality of colors as previously described. The plurality of colors may include a first color assigned to normal and a second color assigned to abnormal. Further, the plurality of colors may include a third color assigned to borderline, a fourth color assigned to critical, and a fifth color assigned to abnormal rhythm. In some embodiments, the color scheme includes changes in hue where a soft color such as blue, purple, and/or green is associated with less severity and a harder color such as yellow, orange, and/or red is associated with more severity. In some embodiments, portions of the waveform 18 assigned normal may be in black or dark blue to contrast the different colors assigned to abnormalities of varying severity. Each segment S1-SN may be associated with the type of condition. In this manner, when the textual information Ti is highlighted relating to one particular type of condition, the segment S1-SN associated with that type of condition may likewise be highlighted and/or otherwise enlarged, while the other segments S1-SN are unchanged. As such, the healthcare provider's attention is drawn to the particular segment S1-SN that formed the basis of the computerized interpretation related in the textual information Ti.

With reference now to FIG. 8, an image 200F is depicted illustrating different features that may be generated on the display 12. For example, in some embodiments, the user interface 14 includes a keypad and the control system 100 (e.g., the processor 104) may be configured to, based on instructions in the memory 106 (i.e., the display guide module 114) or remote storage 150, generate a diagnosis section 202 that receives inputs from the keypad from a healthcare provider related to a clinical diagnosis. More particularly, the healthcare provider may be able to quickly visualize the basis for the computerized interpretation and formulate the clinical diagnosis within the diagnosis section 202 of the image 200A-200J. In still further embodiments, a healthcare provider may be able to interface with the textual information Ti (e.g., via the cursor, a touchscreen, and/or the like) and select an option to incorporate portions of the computerized interpretation into the diagnosis section 202.

With continued reference to FIG. 8, the image 200F generated on the display 12 may further include a notes section 202 for the healthcare provider to enter notes during formulation of the clinical diagnosis, a record section 204, which may include a series of additional ECG 16 or waveforms 18 the same patient (e.g., the patient's history) or other patients. In this manner, the healthcare provider can quickly review numerous ECGs 16. A tool bar 206, with features such as adjusting the values along different axes of the ECG 16 may also be utilized. Further, the image 200F generated on the display 12 may include actual measured values of various heart behaviors in value section 208. The value section 208 may be changed depending on the alignment of the cursor with the waveform 18, a selection of the textual information Ti, and/or the like. The image 200F includes three noteworthy abnormalities communicated by the textual information Ti. These abnormalities include sinus rhythm as shown with segment S1, a right bundle branch block as shown with segment S2, and a left anterior fascicular block as shown with segment S3. The textual information Ti associated with each of these abnormalities is generated in a different color that corresponds to severity. Likewise, the segments S1-S3 associated with each of these abnormalities is generated in the same color as the associated textual information Ti. In the image 200F, the textual information Ti that recites the right bundle branch block or the segment S2 has been selected (e.g., via the user interface). As a result, the segment S2 of the waveform 18 have been enlarged (e.g., thickened).

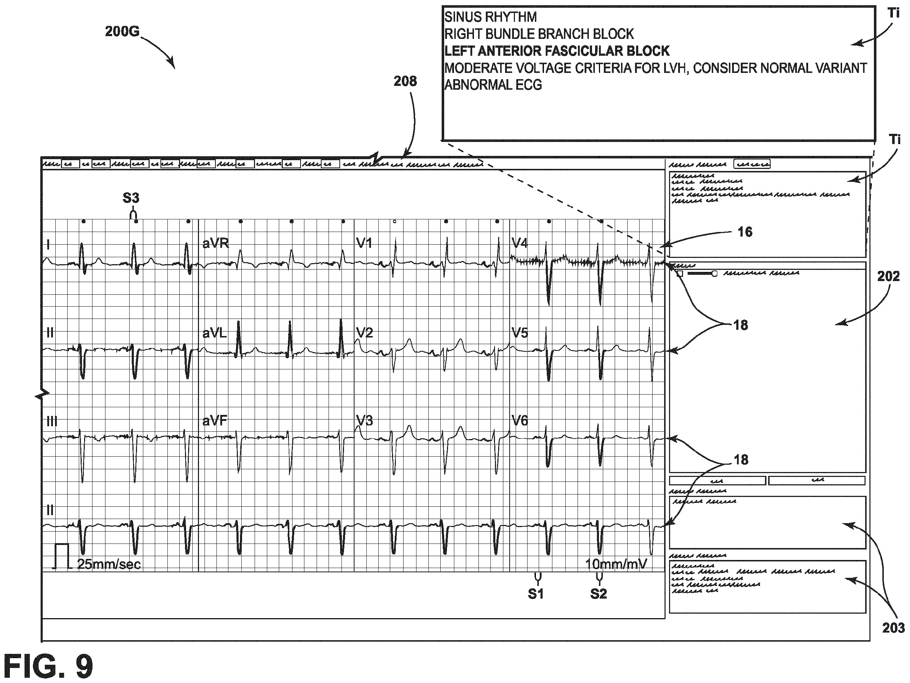

With reference now to FIG. 9, a first partial image 200G generated on the display 12 is illustrated. The first partial image 200G includes the ECG 16 depicted in FIG. 8, but with different features removed for simplicity and a different condition selected. More particularly, in the first partial image 200G, the textual information Ti that recites the left anterior fascicular block or the segment S3 has been selected (e.g., via the user interface). As a result, the segment S3 of the waveform 18 has been enlarged (e.g., thickened).

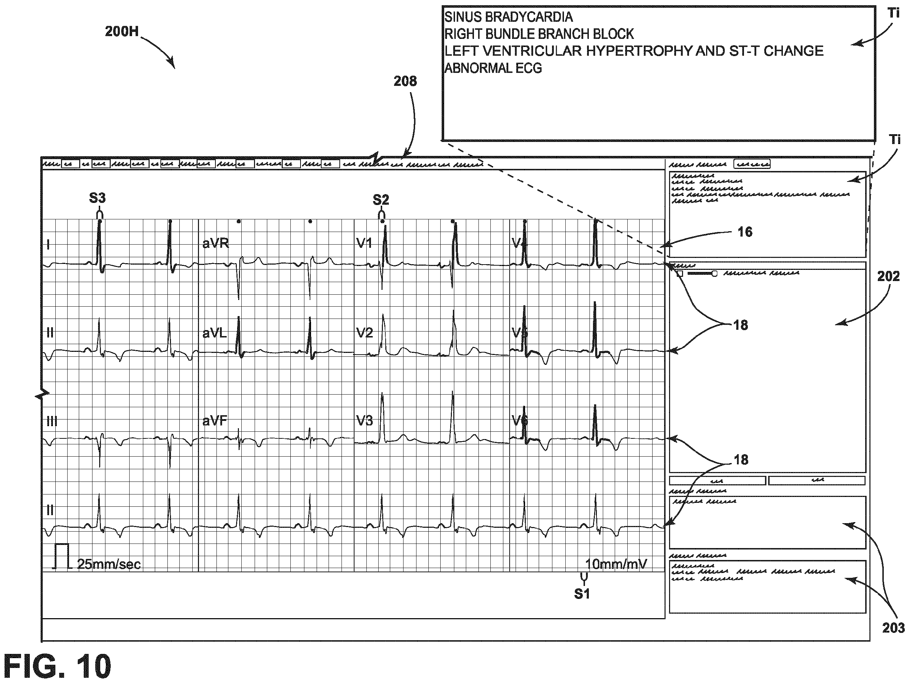

With reference now to FIGS. 10-12, a series of partial images 200H-200J are illustrated. The partial images 200H-200J include three noteworthy abnormalities communicated by the textual information Ti. These abnormalities include sinus bradycardia as shown with segment S1, a right bundle branch block as shown with segment S2, and a left ventricular hypertrophy and ST-T change as shown with segment S3. The textual information Ti associated with each of these abnormalities is generated in a different color that corresponds to severity. Likewise, the segments S1-S3 associated with each of these abnormalities is generated in the same color as the associated textual information Ti.

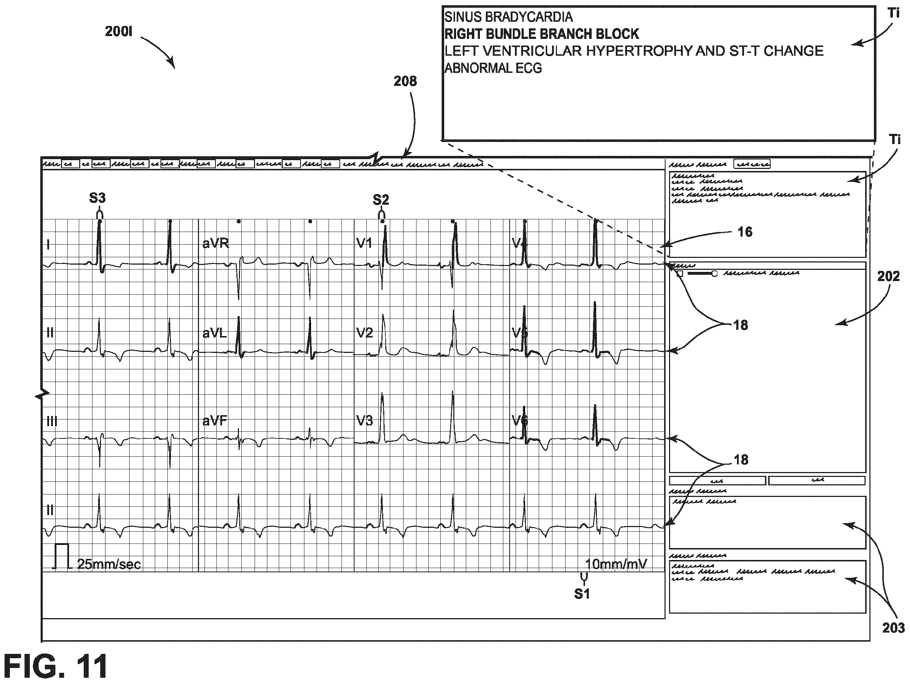

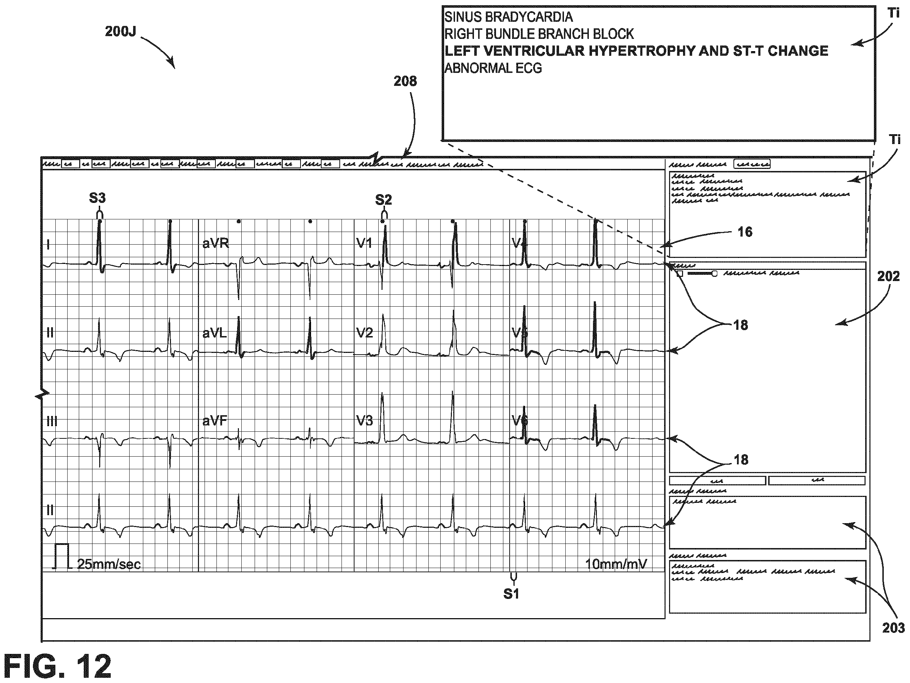

In FIG. 10 a second partial image 200H is illustrated with neither the textual information Ti or the segments selected (e.g., via the user interface). As a result, the segments S1-S3 and the textual information Ti are colored, but not enlarged. In FIG. 11, a third partial image 200I is illustrated where the textual information Ti that recites the right bundle branch block or the segment S2 has been selected (e.g., via the user interface). As a result, the segment S2 of the waveform 18 has been enlarged (e.g., thickened). In FIG. 12, a fourth partial image 200H is illustrated where the textual information Ti that recites the left ventricular hypertrophy and ST-T change or the segment S3 has been selected (e.g., via the user interface). As a result, the segment S3 of the waveform 18 has been enlarged (e.g., thickened).

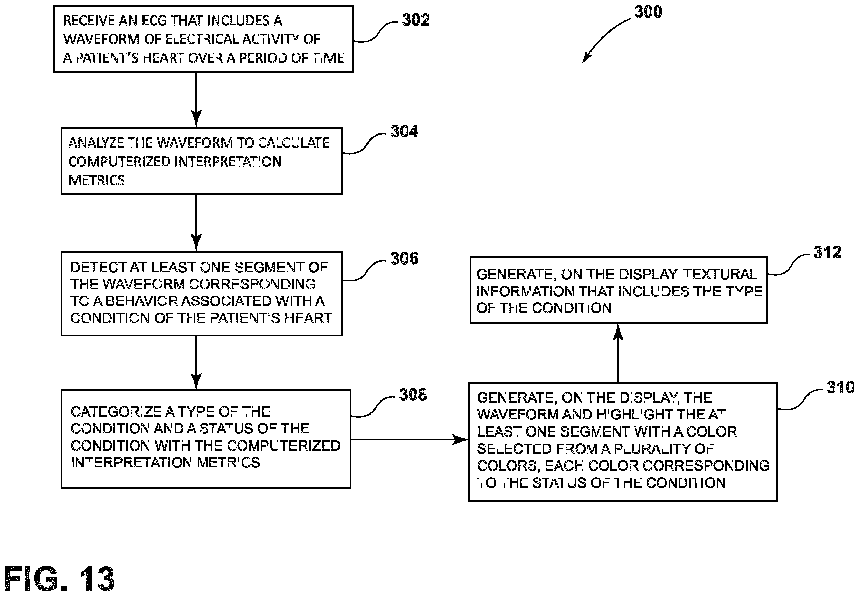

FIG. 13 shows a method 300 of visualizing a computerized interpretation on a display 12 with a user interface 14. At step 302, the method 300 includes receiving an ECG that includes a waveform of electrical activity of a patient's heart over a period of time (e.g., directly from the electrocardiogram device 20, a remote storage 150, and/or the like). At step 304, the method 300 includes analyzing the waveform to calculate computerized interpretation metrics. At step 306, the method 300 includes detecting at least one segment of the waveform corresponding to a behavior associated with a condition of the patient's heart. At step 308, the method 300 includes categorizing a type of condition or the status of the condition with the computerized interpretation metrics. At step 310, the method 300 includes generating, on the display 12, the waveform and highlight the at least one segment with a color selected from a plurality of colors, each color of the plurality of colors corresponding to the type of condition and/or the status of the condition. At step 312, the method 300 includes generating, on the display 12, textual information that includes the type of condition and/or the status of the condition.

Embodiments of the invention can be described with reference to the following numbered clauses, with preferred features laid out in the dependent clauses:
1. An electrocardiogram ("ECG") interpretation system comprising:
   a display;
   a user interface; and
   a processor and a memory, the memory containing instructions that when executed by the processor cause the processor to:
      receive an ECG that includes a waveform of electrical activity of a patient's heart over a period of time;
      analyze the waveform to calculate computerized interpretation metrics;
      detect at least one segment of the waveform corresponding to a behavior associated with a condition behavior associated with a condition of the patient's heart;
      categorize a type of the condition and a status of the condition selected from a group including at least normal and abnormal with the computerized interpretation metrics;
      generate, on the display, the waveform and highlight the at least one segment with a color selected from a plurality of colors, each color of the plurality of colors corresponding to the type of condition or the status of the condition; and
      generate, on the display, textual information that includes the type of the condition and the status of the condition.
2. The ECG interpretation system of clause 1, wherein the textual information further includes information related to portions of the waveform other than the at least one segment.
3. The ECG interpretation system in one of clauses 1 or 2, wherein the textual information that includes the type of the type of condition or the status of the condition is highlighted in the color of the highlight to the at least one segment.
4. The ECG interpretation system of any of the preceding clauses, wherein the highlight on the waveform includes colorizing the at least one segment in the color.
5. The ECG interpretation system in any preceding clauses, wherein the highlight on the textual information includes colorizing a font of the textual information in the color.
6. The ECG interpretation system of any preceding clauses , wherein the highlight on the textual information includes colorizing a background behind a font of the textual information in the color.
7. The ECG interpretation system of any of the preceding clauses, wherein the user interface includes a cursor, and wherein the processor is further caused to:
   detect if the cursor is aligned with the textual information that includes the type of condition or the status of the condition; and
   enlarge the at least one segment on the display.
8. The ECG interpretation system of clause 7, wherein the at least one segment is thickened.
9. The ECG interpretation system of either clause 7 or clause 8, wherein the at least one segment zoomed in on.
10. The ECG interpretation system of any of the preceding clauses, wherein the user interface includes a cursor, and wherein the processor is further caused to:
   detect if the cursor is aligned with the at least one segment; and
   enlarge the textual information that includes the type of condition or the status of the condition.
11. The ECG interpretation system of clause 10, wherein the textual information that includes the type of condition or the status of the condition is bolded.
12. The ECG interpretation system of either clause 10 or clause 11, wherein the textual information that includes the type of condition or the status of the condition is generated in larger font.
13. The ECG interpretation system of any of the preceding clauses, wherein the user interface includes a keypad, and wherein the processor is further caused to:
   generate a diagnosis section;
   receive comments from the keypad related to a clinical diagnosis; and
   generate the comments in the diagnosis section.
14. The ECG interpretation system of any of the preceding clauses , wherein the plurality of colors includes a first color assigned to the status of the condition being normal and a second color assigned to the status of the condition being abnormal.
15. The ECG interpretation system of clause 14, wherein the plurality of colors further includes a third color assigned to the status of the condition being borderline.
16. The ECG interpretation system of clause 15, wherein the plurality of colors further includes a fourth color assigned to the status of the condition being critical.
17. The ECG interpretation system of clause 16, wherein the plurality of colors further includes a fifth color assigned to the type of condition being abnormal rhythm.
18. An electrocardiogram ("ECG") interpretation system comprising:
   a display;
   a user interface; and
   a processor and a memory, the memory containing instructions that when executed by the processor cause the processor to:
      receive an ECG that includes a waveform of electrical activity of a patient's heart over a period of time;
      analyze the waveform to calculate computerized interpretation metrics;
      detect at least one segment of the waveform corresponding to a behavior associated with a condition of the patient's heart;
      categorize a type of the condition and a status of the condition selected from a group including at least normal and abnormal with the computerized interpretation metrics;
      generate, on the display, the waveform; and
      generate, on the display, textual information that includes the type of the abnormality and highlight the textual information with a color selected from a plurality of colors, each color of the plurality of colors corresponding to the severity of the abnormality.
19. The ECG interpretation system of clause 18, wherein the processor is further caused to highlight the at least one segment with the color selected from a plurality of colors.
20. The ECG interpretation system of either clause 18 or clause 19, wherein the highlight on the waveform includes colorizing the at least one segment in the color.
21. The ECG interpretation system in one of clauses 18-20, wherein the highlight on the textual information includes colorizing a font of the textual information in the color.
22. The ECG interpretation system in any one of clauses 18 -21, wherein the user interface includes a cursor, and wherein the processor is further caused to:
   detect if the cursor is aligned with the textual information that includes the type of the abnormality; and
   enlarge the at least one segment on the display.
23. The ECG interpretation system of clause 22, wherein the at least one segment is thickened.
24. The ECG interpretation system of either clause 22 or clause 23, wherein the at least one segment zoomed in on.
25. The ECG interpretation systemin any one of clauses 18-24, wherein the user interface includes a cursor, and wherein the processor is further caused to:
   detect if the cursor is aligned with the at least one segment; and
   enlarge the textual information that includes the type of the abnormality.
26. The ECG interpretation system of clause 25, wherein the textual information that includes the type of the abnormality is bolded.
27. The ECG interpretation system of either clause 25 or clause claim 26, wherein the textual information that includes the type of the abnormality is generated in larger font.
28. The ECG interpretation system in any one of the preceding clauses 18-27, wherein the user interface includes a keypad, and wherein the processor is further caused to:
   generate a diagnosis section;
   receive comments from the keypad related to a clinical diagnosis; and
   generate the comments in the diagnosis section.
29. An electrocardiogram ("ECG") interpretation system comprising:
   a display;
   a user interface; and
   a processor and a memory, the memory containing instructions that when executed by the processor cause the processor to:
      receive an ECG that includes a waveform of electrical activity of a patient's heart over a period of time;
      analyze the waveform to calculate computerized interpretation metrics;
      detect at least one segment of the waveform corresponding to a behavior associated with a condition of the patient's heart;
      categorize a type of the condition and a status of the condition selected from a group including at least normal and abnormal with the computerized interpretation metrics;
      generate, on the display, the waveform and highlight the at least one segment with a color selected from a plurality of colors, each color of the plurality of colors corresponding to the type of condition or the status of the condition; and
      generate, on the display, textual information that includes the type of condition and the status of the condition and highlight the textual information with the color selected from the plurality of colors, each color of the plurality of colors corresponding to the type of condition and the status of the condition.
30. The ECG interpretation system of clause 29, wherein the highlight on the waveform includes colorizing the at least one segment in the color.
31. The ECG interpretation system of either clause 29 or clause 30, wherein the highlight on the textual information includes colorizing a font of the textual information in the color.
32. An electrocardiogram ("ECG") interpretation system comprising:
   a display;
   a user interface; and
   a processor and a memory, the memory containing instructions that when executed by the processor cause the processor to:
      receive an ECG that includes a waveform of electrical activity of a patient's heart over a period of time;
      analyze the waveform to calculate computerized interpretation metrics;
      detect at least one segment of the waveform corresponding to a behavior associated with a condition of the patient's heart;
      categorize a type of the condition and a status of the condition selected from a group including at least normal and abnormal with the computerized interpretation metrics;
      generate, on the display, the waveform and highlight the at least one segment with a first color selected from a plurality of first colors, each first color corresponding to the type of condition or the status of the condition; and
      generate, on the display, textual information that includes the type of condition or the status of the condition and highlight the textual information with the first color selected from the first plurality of colors, each color of the plurality of colors corresponding to the type of condition or the status of the condition.
33. The ECG interpretation system of clauses 32, wherein the processor is further caused to:
   highlight a background behind the at least one segment with a second color selected from a plurality of second colors, each second color corresponding to the type of condition or the status of the condition.
34. The ECG interpretation system of either clause 32 or clause 33, wherein the processor is further caused to:
   highlight a background behind the textual information with the second color selected from the plurality of second colors.
35. An electrocardiogram ("ECG") interpretation system comprising:
   an electrocardiogram device configured to place over a patient's heart that generates a cardiac signal;
   a display;
   a user interface; and
   a processor and a memory, the memory containing instructions that when executed by the processor cause the processor to:
      receive, from the electrocardiogram device, the cardiac signal and generate an ECG that includes a waveform of electrical activity of a patient's heart over a period of time;
      analyze the waveform to calculate computerized interpretation metrics;
      detect at least one segment of the waveform corresponding to a behavior associated with a condition of the patient's heart;
      categorize a type of the condition and a status of the condition selected from a group including at least normal and abnormal with the computerized interpretation metrics;
      generate, on the display, the waveform and highlight the at least one segment with a color selected from a plurality of colors, each color of the plurality of colors corresponding to the type of condition or the status of the condition; and
      generate, on the display, textual information that includes the type of condition or the status of the condition.
36. A method of interpreting an electrocardiogram ("ECG") comprising:
   receiving an ECG that includes a waveform of electrical activity of a patient's heart over a period of time;
   analyzing the waveform to calculate computerized interpretation metrics;
   detecting at least one segment of the waveform corresponding to a behavior associated with a condition of the patient's heart;
   categorizing a type of the condition and a status of the condition selected from a group including at least normal and abnormal with the computerized interpretation metrics;
   generating, on a display, the waveform and highlight the at least one segment with a color selected from a plurality of colors, each color of the plurality of colors corresponding to the type of condition or the status of the condition; and
   generating, on the display, textual information that includes the type of the-condition and the status of the condition.
37. The method of clause 36, further including highlighting the textual information that includes the type of the condition and the status of the condition with the color.
38. The method of clause 36 or clause 37, further including colorizing the at least one segment in the color.
39. The method of any one of clauses 36 to 38, further including colorizing a font of the textual information in the color.
40. The method of any one of clauses 36 to 39, further including colorizing a background behind a font of the textual information in the color.
41. The method of any one of clauses 36 to 40, further including thickening the at least one segment.
42. The method of any one of clauses 36 to 41, further including zooming in on the at least one segment.

## Claims

1. An electrocardiogram ("ECG") interpretation system (10) comprising:
a display (12);
a user interface (14); and
a processor (104) and a memory (106), the memory (106) containing instructions that when executed by the processor (104) cause the processor (104) to:
receive an ECG (16) that includes a waveform (18) of electrical activity of a patient's heart over a period of time;
analyze the waveform (18) to calculate computerized interpretation metrics;
detect at least one segment (S1-SN) of the waveform (18) corresponding to a behavior associated with a condition associated with a condition of the patient's heart;
categorize a type of the condition and a status of the condition selected from a group including at least normal and abnormal with the computerized interpretation metrics;
generate, on the display (12), the waveform (18) and highlight the at least one segment (S1-SN) with a color selected from a plurality of colors, each color of the plurality of colors corresponding to the type of condition or the status of the condition; and
generate, on the display (12), textual information (Ti) that includes the type of the condition or the status of the condition.

2. The ECG interpretation system (10) of claim 1, wherein the textual information (Ti) further includes information related to portions of the waveform (18) other than the at least one segment (S1-SN).

3. The ECG interpretation system (10) of either claim 1 or claim 2, wherein the textual information (Ti) that includes the type of condition is highlighted in the color of the highlight to the at least one segment (S1-SN).

4. The ECG interpretation system (10) of any preceding claim, wherein the highlight on the waveform (18) includes colorization of the at least one segment (S1-SN) in the color.

5. The ECG interpretation system (10) of any preceding claim, wherein the highlight on the textual information (Ti) includes colorization of a font of the textual information (Ti) in the color.

6. The ECG interpretation system (10) of claim 4, wherein the highlight on the textual information (Ti) includes colorizing a background behind a font of the textual information (Ti) in the color.

7. The ECG interpretation system (10) of any preceding claim, wherein the processor (104) is further caused to:
detect if a user interacts with the textual information (Ti) that includes the type of condition or the status of the condition; and
enlarge the at least one segment (S1-SN) on the display (12).

8. The ECG interpretation system (10) of claim 7, wherein the at least one segment (S1-SN) is thickened.

9. The ECG interpretation system (10) of either claim 7 or claim 8, wherein the at least one segment (S1-SN) zoomed in on.

10. The ECG interpretation system (10) of any proceeding claim, wherein the processor (104) is further caused to:
detect if a user interacts with the at least one segment (S1-SN); and
enlarge the textual information (Ti) that includes the type of condition or the status of the condition.

11. The ECG interpretation system (10) of any preceding claim, wherein the textual information (Ti) that includes the type of condition also includes the status of the condition.

12. The ECG interpretation system (10) of claim 11, wherein the textual information (Ti) that includes the type of condition and the status of the condition is generated in larger font.

13. The ECG interpretation system (10) of any preceding claim, wherein the user interface (14) includes a keypad, and wherein the processor (104) is further caused to:
generate a diagnosis section (202);
receive comments from the keypad related to a clinical diagnosis; and
generate the comments in the diagnosis section (202).

14. The ECG interpretation system (10) of any preceding claim, wherein the plurality of colors includes a first color assigned to the status of the condition being normal and a second color assigned to the status of the condition being abnormal.

15. The ECG interpretation system (10) of claim 14, wherein the plurality of colors further includes a third color assigned to the status of the condition being borderline, and a fourth color assigned to the status of the condition being critical.
